# EUROPEAN PATENT SPECIFICATION

(11) **EP 1 846 093 B1**
(45) Date of publication and mention of the grant of the patent: **22.05.2013**
(21) Application number: 06733938.2
(22) Date of filing: 26.01.2006
(51) Int. Cl.: A61N 1/372, A61N 1/37

(54) **SYSTEM FOR OPERATING AN IMPLANTABLE MEDICAL DEVICE IN A DISRUPTIVE ENERGY FIELD**
SYSTEM ZUM BETRIEB EINER IMPLANTIERBAREN MEDIZINISCHEN VORRICHTUNG IN EINEM DISRUPTIVEN ENERGIEFELD
SYSTEME POUR L'EXPLOITATION DE DISPOSITIF MEDICAL IMPLANTABLE DANS UN CHAMP D'ENERGIE DISRUPTIVE

(30) Priority: 26.01.2005 US 43291
(43) Date of publication of application: 24.10.2007
(73) Proprietor: Medtronic, Inc., Minneapolis, MN 55432-5604 (US)
(72) Inventor: NELSON, Shannon, D., Minneapolis, Minnesota 55409 (US); HOOPER, Dan, A., Oak Grove, Minnesota 55011 (US); ERICSON, Eric, B., Maple Grove, Minnesota 55369 (US); CRANDALL, Kathleen, U., Champlin, Minnesota 55316 (US); SWANBERG, Paul, R., Edina, Minnesota 55435 (US); HAERLE, Mark L., Bloomington, MN 55438 (US); ECHOLS, Lori L., Parker, CO 80134 (US)
(74) Representative: Hughes, Andrea Michelle
(86) International application number: PCT/US2006/002827
(87) International publication number: WO 2006/081374

(56) References cited:
- US-A1- 2002 133 216
- US-A1- 2003 083 570
- US-A1- 2003 144 705
- US-B1- 6 209 764

## Description

The present invention relates to implantable medical devices, and more particularly to a system for operating an implantable medical device (IMD) in a disruptive energy field.

Advancements in medical technology have led to the use of devices for imaging and for therapy that involve exposing a patient to energy fields that may be disruptive to implantable medical devices. For example, magnetic resonance imaging (MRI) generates cross-sectional images of a human body using nuclear magnetic resonance (NMR). The MRI process begins with positioning the body to be imaged in a strong, uniform magnetic field, which polarizes the nuclear magnetic moments of protons within hydrogen molecules in the body by forcing their spins into one of two possible orientations. Then an appropriately polarized radio-frequency field, applied at resonant frequency, forces spin transitions between these orientations. The spin transitions create a signal, an NMR phenomenon, which can be detected by a receiving coil.

Shortwave diathermy, microwave diathermy, ultrasound diathermy, and the like have been shown to provide therapeutic benefits to patients, such as to relieve pain, stiffness, and muscle spasms; to reduce joint contractures; to reduce swelling and pain after surgery; and to promote wound healing. Generally, energy (e.g., shortwave energy, microwave energy, or ultrasound energy) is directed into a localized area of the patient's body. Energy fields are also developed in other beneficial procedures such as cautery during a surgical procedure.

Traditionally, the use of the above-described devices have been discouraged for patients with implanted medical devices, because the field produced by MRI, diathermy, or cautery devices may interfere with or affect the performance of IMDs. The energy fields generated during the MRI or diathermy processes may induce a voltage on the leads that can be dissipated within tissue adjacent lead electrodes. This may cause tissue damage if the voltage attains high levels. Additionally, a sudden burst of radio-frequency energy can deliver a current pulse that may induce a tachycardia or fibrillation. Gradient magnetic fields can have similar detrimental effects. Energy fields can affect sensed signals resulting in inappropriately delivered pacing and/or shocks.

US 2003/0144705 teaches a pacing system having an interference state pacing mode and pacing rate when EMI is detected.

The present invention is a system for operating an implantable medical device in a disruptive energy field generated during an MRI scan. The system includes an IMD capable of being operated in a normal operating mode an exposure mode and a programmer having an application executing thereon that performs an MRI compatible certification process to perform a number of safety checks to ensure that the IMD is MRI compatible before enabling operation in the exposure mode.
FIG. 1 shows an MRI environment.
FIG. 2 is a block diagram illustrating an IMD.
FIG. 3 is astate diagram of the operating modes of the IMD.
FIG. 4 is a block diagram of a programmer.
FIG. 5 is a flow diagram illustrating the process taken by a caregiver and a radiologist to perform an MRI scan on a patient having the IMD.
FIG. 6 is a flow chart illustrating the programmer application.
FIG. 7 is a screen shot of an MRI dialog screen of the programmer application.

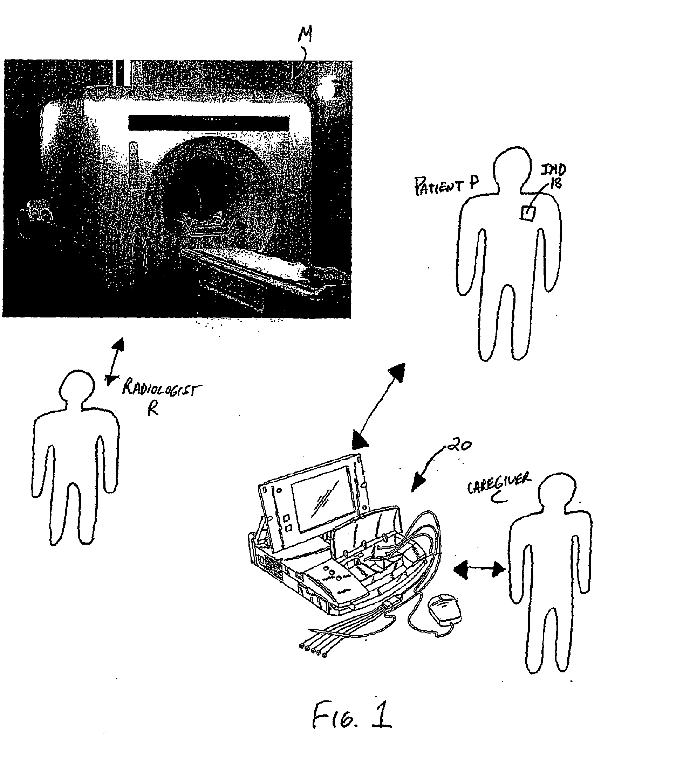

FIG. 1 shows an MRI environment that includes MRI scanner M, radiologist R, patient P, and caregiver C. IMD 18 is implanted in patient P to provide a therapy, such as pacing or defibrillation of the hear, drug delivery, or nerve stimulation. External programming unit (programme) 20 can communicate with IMD 18.

MRI scanner M is a magnetic resonance imaging system which generates cross-sectional images of human body by using nuclear magnetic resonance (NMR). Although the present invention is described with reference to MRI system M, it is recognized that the present invention will also provide benefits for use with other systems that radiate energy or produce magnetic, electromagnetic, and electric fields such as shortwave or microwave diathermy.

IMD 18 is an implantable medical device which is implanted in patient P to provide therapies, and is operable in an MRI environment. IMD 18 is capable of operating in two distinct modes: a normal operating mode for normal device opetation, and an MRI exposure mode for operating during an MRI scan. IMD 18 also records useful data related to diagnosis, therapy delivery, or device operation and performance, and provides self-monitoring of system operation (such as lead impedance data, high-voltage capacitor charge times, battery capacity, etc).

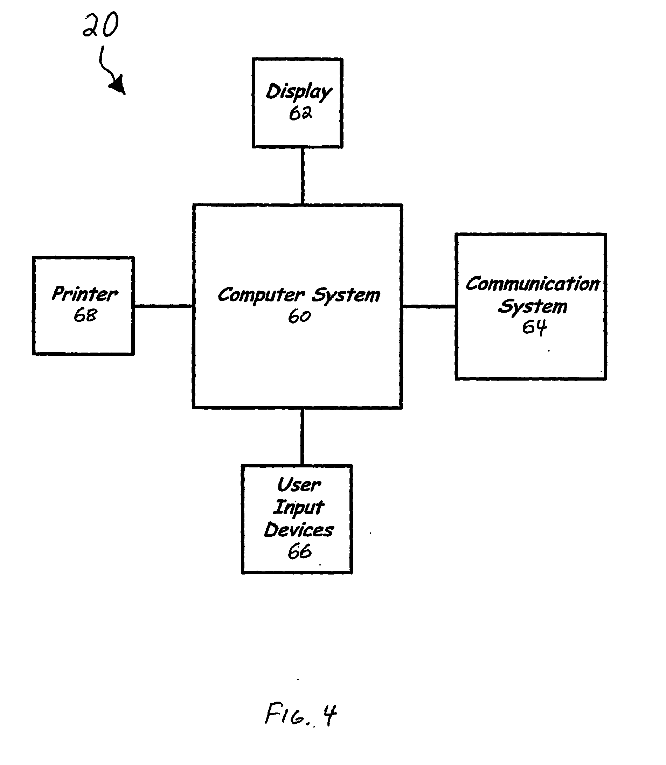

Programmer 20 is an external programming unit that is capable of bi-directional data communication with IMD 18. Programmer 20 includes a user interface through which caregiver C may interact with programmer 20 to select the desired settings to be programmed in IMD 18. Using programmer 20, caregiver C can manually program IMD 18 from the normal operating mode into the MRI exposure mode. Although programmer 20 is described with reference to a portable external programming unit, it is recognized that any device capable of bi-directional data communication with IMD 18 may be utilized. Programmer 20 is described in more detail with reference to FIG. 4.

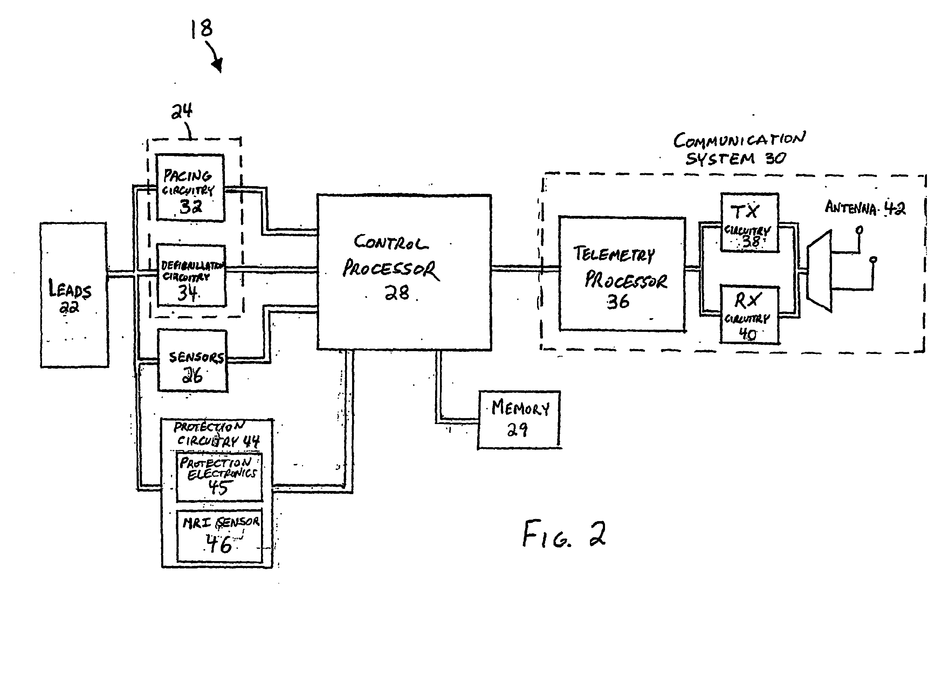

FIG. 2 is a block diagram illustrating IMD 18. Although it is recognized that IMD 18 may be any type of implantable medical device, a specific example will now be provided in which IMD 18 is an implantable cardioverter defibrillator. IMD 18 includes leads 22, therapy delivery circuitry 24, sensors 26, control processor 28, memory 29, communication system 30, and protection circuitry 44.

Leads 22 provide a conductive path between the heart and therapy delivery circuitry 24 and sensors 26. Leads 22 may be a single lead, or multiple leads surrounded by an insulating sleeve. Therapy delivery circuitry 24 includes pacing circuitry 32 and defibrillation circuitry 34 that enable IMD 18 to provide pacing and defibrillation therapies to patient P through leads 22. Sensors 26 detect the electrical signals of the heart, and provide a representation of the electrical signals to control processor 28.

Control processor 28 controls the overall operation of IMD 18. Control processor 28 utilizes sensors 26 to monitor the electrical signals of the heart and processes sensed signals to determine when therapy delivery is desirable. At the appropriate time, control processor 28 activates therapy delivery circuitry 24 to provide the appropriate therapy for patient P. Control processor 28 communicates with programmer 20 through communication system 30.

Memory 29 includes read only memory (ROM) and random access memory (RAM) that store data and instructions for use by control processor 28. Specifically, memory 29 includes data and instructions relating to a mode adjustment feature of IMD 18. The mode adjustment feature enables IMD 18 to operate in the normal operating mode during normal device operation and in the MRI exposure mode when exposed to a disruptive field. The MRI exposure mode enables selected operation of IMD 18 to continue and provides adjustment features to operate in the disruptive energy field. The present invention provides for automatic mode adjustment through field sensors, or manual adjustment by caregiver C through programmer 20.

Communication system 30 includes telemetry processor 36, transmitter circuitry 38, receiver circuitry 40, and antenna 42. Communication system 30 is a wireless communication system utilizing radio frequency electromagnetic waves. However, it is recognized that any communication system capable of bi-directional communication with IMD 18 may also be used.

Protection circuitry 44 includes protection electronics 45 and disruptive field sensor 46. Protection electronics 45 protect IMD 18 from voltages that are induced on leads 22 by gradient fields and RF energy produced by MRI scanner M. Disruptive field sensor 46 is a magnetic, electric, or electromagnetic sensor that enables IMD 18 to detect the start and end of a disruptive energy field generated by an MRI scan. Examples of protection circuitry 44 are disclosed, for example, in U.S. Patent Application No. 10/059,589, filed January 29, 2002. One example of disruptive field sensor 46 is a Hall effect sensor. When disruptive field sensor 46 receives a signal indicative of a disruptive energy field, it sends a signal to control processor 28 to alert control processor 28 to the presence of the disruptive energy field.

In another embodiment, IMD 18 utilizes sensors 26, to detect the start and end of an MRI scan. In this embodiment, sensors 26 (which are used to detect intrinsic intracardiac signals enable IMD 18 to detect the presence of the disruptive energy field. Specifically, the detection is done by sensing a voltage induced on leads 22 as a result of the disruptive energy field.

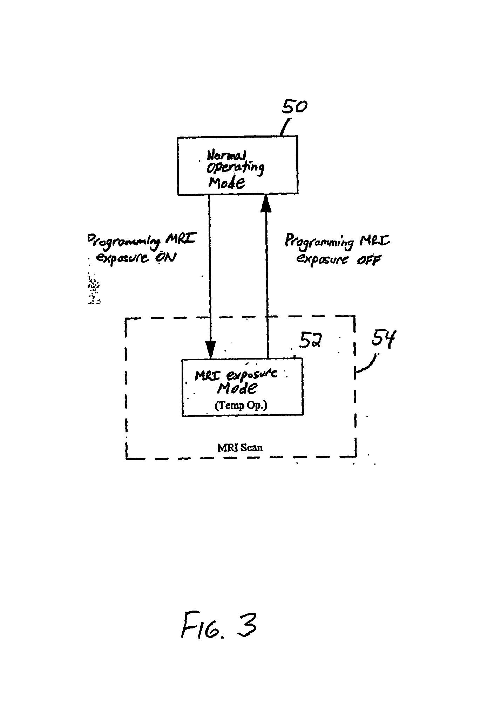

FIG. 3 is a state diagram of the operating modes of IMD 18. The state diagram includes normal operating mode 50 (also known as a permanent programming mode) and MRI exposure mode 52 for use during MRI scan 54. Typically, IMD 18 operates in normal operating mode 50. In this mode all desired functionality of IMD 18 is available, such as event sensing, therapy delivery, and data logging. Prior to MRI scan 54, IMD 18 is adjusted from normal operating mode 50 to MRI exposure mode 52 by initiating a temporary operation in IMD 18. The temporary operation puts IMD 18 into MRI exposure mode 52 by disabling or suspending a number of device operations, and enabling a set of MRI parameters. The temporary operation does this by overriding normal operating mode 50 with the temporary MRI exposure mode 52. After MRI scan 54 has been completed, IMD 18 returns to normal operating mode 50 and resumes normal device operation.

When IMD 18 enters the MRI exposure mode, IMD 18 operates differently than it does during normal device operation. Some of the changes include: recording a time and date stamp, initiating a temporary operation to provide a set of operational parameters, utilizing a temporary operation to prevent certain device operations from occurring, and disabling device operations that could have detrimental effects or cause confusion to the caregiver. Each of these are described in more detail below.

A time and date stamp in memory 29 provides an indication in the stored data that a disruptive field was encountered and that IMD 18 initiated operation in the MRI exposure mode at the recorded time. When stored data is later reviewed, the time and date stamp will indicate to caregiver C the time and date in which IMD 18 encountered the disruptive field and began operating in the MRI exposure mode. In addition, a time and date stamp can also be stored to indicate the time and date in which the disruptive field ended and IMD 18 returned to normal operation.

Various operational parameters can be set upon the initiation of the MRI exposure mode and the temporary operation including: fixed ventricular and atrial amplitude (e.g. 3V), fixed ventricular and atrial pulse width (e.g. 0.4 ms), pacing mode to a caregiver-selectable MRI exposure mode including an asynchronous pacing mode or a sensing only mode, and pacing rate to a caregiver selectable MRI pacing rate. Enabling a fixed asynchronous pacing mode ensures that IMD 18 will continue providing the necessary therapies to patient P, even in the presence of the disruptive energy field. Examples of acceptable pacing modes include: dual-chamber asynchronous pacing mode (DOO), ventricular asynchronous pacing mode (VOO), atrial asynchronous pacing mode (AOO), and sense only mode (ODO) with sensing disabled for patients that do not require continuous pacing.

The temporary operation in MRI exposure mode can also be used to prevent certain device operations from occurring, particularly those that may function incorrectly when IMD 18 is exposed to a disruptive energy field. These suspended operations may include: atrial tachycardia (AT) detection, atrial fibrillation (AF) detection, ventricular tachycardia (VT) monitoring, AT/AF therapies, tripping of Elective Replacement Indicator (ERI), P+R wave measurements, battery measurements, and sub-threshold lead impedance measurements.

Additionally, the following device operations can be suspended to avoid confusing the caregiver, and to enhance the delivery of effective therapy: sub-threshold lead impedance measures, P+R wave measurements, collection of diagnostic data (including episodes, long term clinical trends, device trends, charge time counter, histograms, PVC counter, brady pace counter, brady event sequence, patient alert, ERI indicator, excessive charge time condition, and morphology history buffer), tripping of ERI, and magnet mode. Similarly, the initiation of the temporary operation disables the following counters to avoid incorrectly recording induced signals as cardiac events: ventricular short interval counter, atrial short interval counter, single premature beat counter, premature beat run counter, lifetime atrial premature atrial contractions counter, and lifetime atrial premature atrial contractions run counter.

One of the ways of enabling the MRI exposure mode in IMD 18 is by utilizing the user interface of programmer 20 to select the desired operating mode and operational parameters, and instruct programmer 20 to program IMD 18 into that mode. To ensure that IMD 18 is MRI compatible when programmed into the MRI exposure mode, IMD 18 and programmer 20 include a number of interlocks. The interlocks provide a method of ensuring that certain conditions are met before IMD 18 can be programmed into MRI exposure mode 52. Implementation of the interlocks in programmer 20 is described below with reference to the programmer user interface. Interlocks may include rules such as: (i)

MRI exposure mode can only be enabled if the MRI exposure pacing mode is programmed to an asynchronous pacing mode (DOO, VOO, or AOO) for pacemaker dependent patients or ODO for non-pacemaker dependent patients in order to undergo an MRI scan, (ii) MRI exposure mode can not be turned ON if ERI has been met, (iii) MRI exposure mode can only be turned ON if the impedance of the atrial and ventricular leads is below 1500 ohms, and (iv) MRI exposure mode should only be turned ON if the atrial and ventricular permanent pacing outputs are greater than 2.5V or 0.4 ms when the MRI exposure pacing mode is an asynchronous pacing mode (DOO, VOO, or AOO).

FIG. 4 is a block diagram of programmer 20, which includes computer system 60, display 62, communication system 64, user input devices 66, and printer 68. Computer system 60 controls the overall operation of programmer 20. Computer system 60 includes memory to store application and configuration data. Display 62 is, for example, a liquid-crystal or electroluminescent display. Communication system 64 is a system capable of bi-directional communication with IMD 18 such as a wireless RF communication system, or a magnetic coil communication system. User input devices 66 include keys or switches on the outside of programmer 20 that are initiated by the user to select options or perform designated functions, such as an emergency key that enables caregiver C to quickly access an emergency programming function. In addition, user input devices may include a keyboard, mouse, stylus, touchpad, trackball, or any other user input device. A stylus can be used, for example, to input information when pressed against the appropriate locations of display 62. Printer 68 enables programmer 20 to print out reports, data, or graphs on paper.

Programmer 20 enables caregiver C to program desired settings or modes within IMD 18. Programmer 20 includes software that executes on computer system 60 and provides a user interface on display 62. The user interface provides information and options to caregiver C to allow caregiver C to uplink to IMD 18 and collect device and/or patient related information, as well as to select desired modes and settings to be programmed into IMD 18. Once the appropriate settings have been selected by caregiver C, programmer 20 communicates with IMD 18 through communication system 64. Communication system 64 is configured to communicate with communication system 30 of IMD 18 to enable the transfer of instructions from programmer 20 to IMD 18, as well as the transfer of device data from IMD 18 to programmer 20.

Before describing the specific implementation details of the mode adjustment, it is useful to understand the general process taken by caregiver C and radiologist R to perform an MRI scan on a patient having MRI compatible IMD 18 of the present invention.

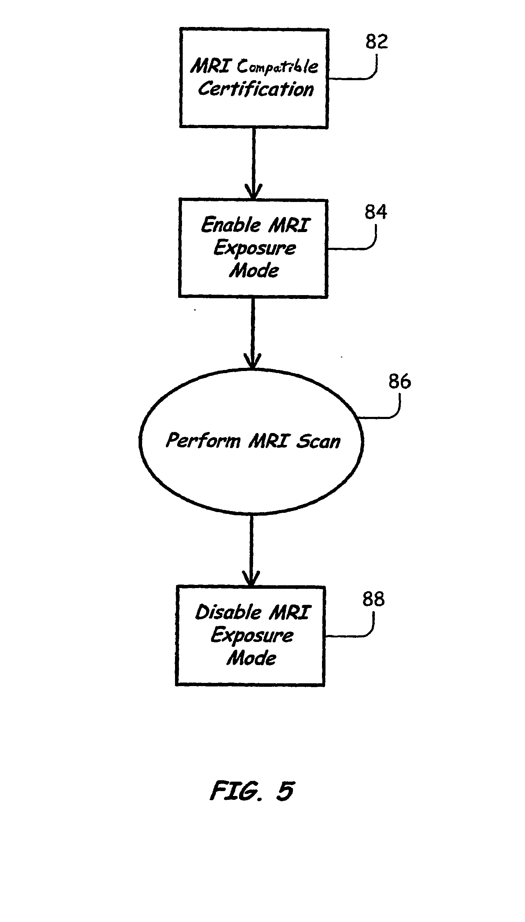

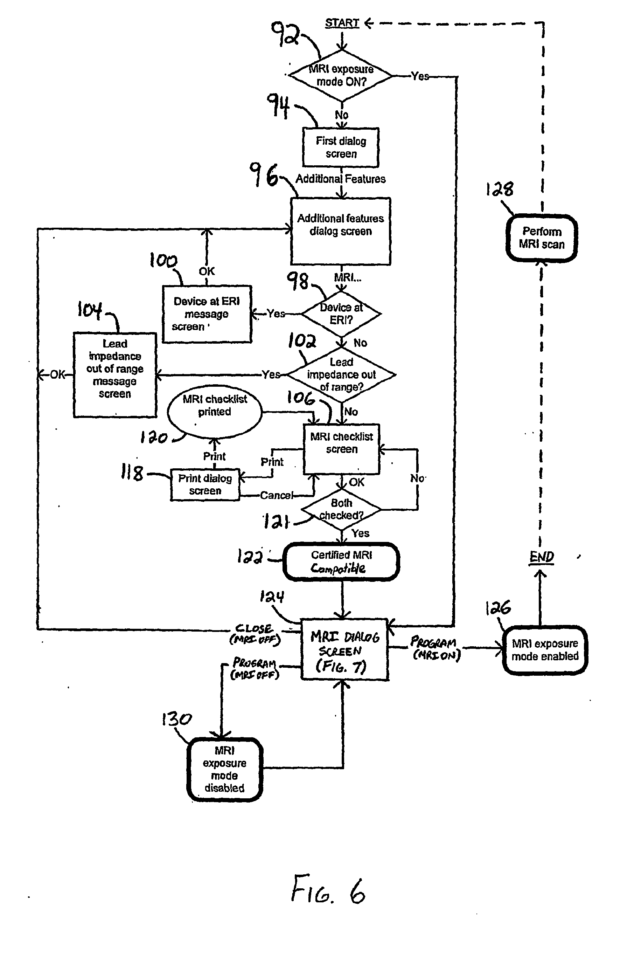

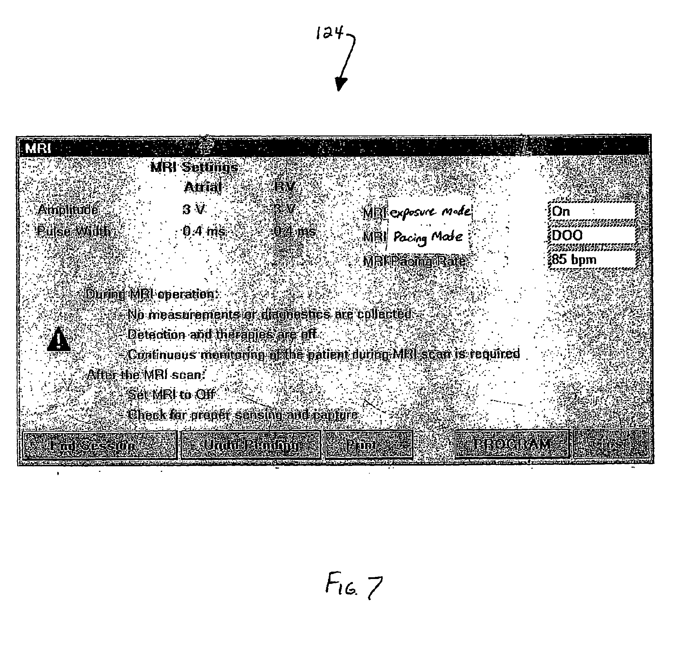

FIG. 5 is a flow diagram illustrating the process taken by caregiver C and radiologist R to perform an MRI scan on patient P having IMD 18. Prior to performing an MRI scan, MRI compatible certification 82 is performed to ensure that both patient P and IMD 18 are in condition to be scanned. This process is described in more detail with reference to FIGS. 6, and 7 below. MRI compatible certification 82 includes ensuring that the specific IMD and leads implanted in patient P are MRI compatible. This involves determining what type of IMD and leads are implanted in patient P and determining whether the manufacturer of these products has designated them to be MRI compatible. In addition, the system is not MRI compatible if it includes any of the following: abandoned leads, lead extenders, or any device or product not designed or designated as MRI compatible. If caregiver C determines that the IMD or leads within patient P are not MRI compatible, no MRI procedure will be performed.

In another embodiment, MRI compatible certification 82 is performed automatically by IMD 18 or programmer 20 by determining if IMD 18 includes an MRI exposure mode, and if it does, concluding that IMD 18 is MRI compatible. Alternatively, a look-up table could be provided in programmer 20 to determine whether the device model and software version is MRI compatible. Other safety checks are also capable of being performed in MRI compatible certification 82, as described below. These include verifying that the device is not at ERI, and that the lead impedance is not out of range. After MRI compatible certification 82 is completed, the MRI exposure mode is enabled (step 84). As previously described this may be done automatically by IMD 18 or manually by caregiver C through programmer 20. If IMD 18 includes an MRI sensor, and IMD 18 is configured to automatically start when it detects an MRI scan, then the step of enabling the MRI exposure mode (step 84) is performed automatically by IMD 18 when the MRI scan begins (step 86). At the end of the MRI scan (step 86), IMD 18 automatically detects that the MRI scan has completed, and IMD 18 returns to the normal operating mode, thereby disabling the MRI exposure mode (step 88).

If IMD 18 is not configured with an MRI sensor or if caregiver C prefers to enable the MRI exposure mode manually, then caregiver C utilizes programmer 20 (as described below) to enable the MRI exposure mode in IMD 18. After the MRI exposure mode has been enabled (step 84), radiologist R performs the MRI scan (step 86), following all necessary safety procedures. After the MRI scan is complete (step 86) caregiver C again utilizes programmer 20 to disable the MRI exposure mode (step 88) in IMD 18 to return IMD 18 to the normal operating mode.

Programmer 20 includes software and/or firmware (referred to generally as the application that enables caregiver C to manually enable and disable the MRI exposure mode in IMD 18 through a user interface. Caregiver C interacts with the user interface to perform the steps necessary to prepare IMD 18 to be scanned, and after the scan, to return IMD 18 to the normal operating mode. The programmer application will now be described in more detail.

FIG. 6 is a flow chart illustrating the programmer application. The application executing on programmer 20 begins (see START) by determining whether the MRI exposure mode is already ON in IMD 18 (step 92). This first decision step enables programmer 20 to determine whether IMD 18 is currently programmed in the MRI exposure mode. If so, the application bypasses the normal dialog screen, and immediately presents the MRI dialog screen. If the MRI exposure mode is not ON, first dialog screen 94 is displayed. First dialog screen 94 may include diagnostic, therapeutic, or patient related data, and provides a series of options. To begin an MRI scan, caregiver C navigates through the screens to locate the option to enable MRI-related functionality. In this embodiment, the MRI option is selected through an additional features dialog screen which is accessed by selecting the "Additional Features" option. From here, caregiver C can directly select the "MRI ..." option to begin the process of enabling the MRI exposure mode.

Once the MRI option has been selected, the application begins the MRI compatible certification process (82 of FIG. 5). The MRI compatible certification process performs a number of safety checks to ensure that IMD 18 is MRI compatible. The application checks first to see whether an ERI condition has been met in IMD 18 (step 98). If IMD 18 is at ERI, the application recognizes that it may not be safe to scan IMD 18, and therefore displays an error message (device at ERI message screen 100) indicating that IMD 18 is at ERr. Recognizing that IMD 18 should not be placed in a disruptive energy field while at ERI, the application returns to the additional features dialog screen, thereby terminating the adjustment into the MRI exposure mode.

If IMD 18 is not at ERI, the application then proceeds to check whether the impedance of any leads is out of range (step 102). Lead impedance is important because an out of range lead impedance could indicate a break or short in a lead. To determine the lead impedance, the application checks the last recorded lead impedance measurements within IMD 18, or in an alternate embodiment, it instructs IMD 18 to perform a lead impedance check. The measured values are compared to a predetermined range of values (for example, a range of 0 to 1500 ohms). If the measured values are outside of this range, the application displays an error message (lead impedance out of range message screen 104). The error message indicates to caregiver C that the device is not MRI compatible because the impedance of one or more bipolar leads exceeds safety requirements or cannot be verified. Once caregiver C selects OK, the application returns to additional features dialog screen 96 and will not allow IMD 18 to be programmed into the MRI exposure mode.

Provided that IMD 18 is not at ERI and all lead measurements are within the predetermined range, MRI checklist 106 is displayed. MRI checklist 106 provides information to caregiver C to instruct on what steps need to be taken prior to exposure of IMD 18. Checklist 106 is divided into two sections, the first section providing information for caregiver C, and the second section providing information to be given to radiologist R. An option is provided to allow the information to be printed.

Checklist 106 instructs caregiver C to verify that: IMD 18 has been implanted for at least six weeks, IMD 18 was implanted in the pectoral region, no additional active IMD are present, leads are electrically intact and MRI compatible, no abandoned leads or wires are present, and no lead extenders or adapters are present. Checklist 106 also instructs radiologist R to verify that: the MRI scanner is used within Normal Operating Mode limits, and that the MRI scanner operates at 1.5 or 3.0 Tesla. The Normal Operating Mode limits are defined by the International Electrotechnical Commission (IEC) 60601-2-33 standard. In addition, limitations may be placed on the maximum length of a scan (such as 30 minutes), a minimum cool down period between scans (such as 10 minutes), RF power limits, types of MRI coils that may be used, limitations on localized scanning of the region of the implantable device, maximum gradient magnetic fields (such as the default value from the 2001-7-30 version of the IEC 60601-2-33, clause 1.102.2.2 b, which is less than or equal to 16T/s * (1+0,36 / tseff), where tseff is the effective stimulus duration of any period of the monotonic increasing or decreasing gradient, used to describe its limits for cardiac or peripheral nerve stimulation). Furthermore, checklist 106 may suggest additional safety monitoring devices or steps be provided during the MRI scan for both non-pacemaker dependent patients and pacemaker dependent patients.

Monitoring may include providing electrocardiography, pulse oximetry, or non-invasive blood pressure measurements.

After each of these two steps are complete, caregiver C selects the check boxes indicating that the necessary steps have been completed, and selects OK. The application will not continue until both check boxes have been selected. Completion of checklist 106 concludes the MRI compatible certification process (step 122). If caregiver C decides not to continue, he or she selects cancel to exit checklist 106 and returns to additional features dialog screen 96.

After completion of checklist 106, MRI dialog screen 124 is displayed (as shown in FIG. 7). MRI dialog screen 124 is the main menu of the MRI portion of the application that enables caregiver C to select all desired options related to the MRI exposure mode, including enabling or disabling the MRI exposure mode. MRI dialog screen 124 includes a display of current settings of IMD 18, a list of MRI exposure mode options, and instructions. The display of current settings includes data such as atrial and ventricular amplitude and pulse-width settings. MRI exposure mode options include an MRI exposure mode on/off option, an MRI pacing mode option, and an MRI pacing rate option.

The instructions provide information to remind caregiver C of what the MRI exposure mode will do, and what steps need to be taken prior to, during, and after the MRI scan.

To program IMD 18 into the MRI exposure mode prior to an MRI scan, caregiver C selects ON from the MRI exposure mode on/off option. Next, the desired MRI pacing mode is selected. Finally, the desired MRI pacing rate is chosen if the MRI pacing mode is an asynchronous pacing mode. No pacing rate is necessary if pacing will not be utilized during the scan. Caregiver C then selects Program to enable the MRI exposure mode (step 126) and settings in IMD 18. IMD 18 is now able to be exposed to the disruptive fields generated by the MRI scan (step 128).

After the MRI scan is completed (step 128), the programmer application restarts (START). Programmer 20 communicates with IMD 18 and determines that the MRI exposure mode is on (step 92), and therefore immediately displays the MRI dialog screen 124 to caregiver C. At the MRI dialog screen 124, Caregiver C selects OFF from the MRI exposure mode on/off option, and selects Program to disable the MRI exposure mode (step 130) and return IMD 18 to the normal operating mode. In an alternate embodiment, programmer 20 includes a one-click option to quickly disable the MRI exposure mode.

Caregiver C then selects Close to exit the MRI-telated screens and return to additional features dialog screen 96.

The system of the present invention enables a patient with an IMD to obtain the benefits of an MRI scan or to be exposed to other electrical, magnetic, or electromagnetic fields, while ensuring that the IMD will continue to operate as desired.

The MRI exposure mode of the implantable medical device reduces or eliminates the risks of obtaining an MRI scan, and therefore provides great benefics. An MRI sensor in an implantable medical device allows the MRI exposure to be enable and disabled automatically. Alternatively, a programmer is used by a caregiver to enable, disable, and adjust the MRI exposure mode settings. The user interfaces assists the caregiver in performing the necessary steps, and guides the caregiver through the process with simple and easy to follow screens, instruction, and printouts. Thus, the present invention is an improvement in the art which will provide many benefits to patients, caregivers, and radiologists alike.

Although the present invention has been described with reference to preferred embodiments, workers skilled in the art will recognize that changes may be made in form and detail without departing from the scope of the invention. In particular, the present invention is described with refence to and MRI scan. It is recognized that the present invention is equally applicable when implantable medical device is exposed to magnetic, electromagnetic, or electric fields or waves. Hence, terms such as MRI scan, and MRI compatible should be understood to include any other device or procedure that utilizes magnetic, electromagnetic, electric, or gradient fields or waves, or any combinations thereof. In addition, the present invention has been described with specific roles for a caregiver and a radiologist. It is racognized that the present invention is equally applicable for others who desire to perform these tasks.

## Claims

1. A system comprising:
an implantable medical device operable in a disruptive energy field, the implantable medical device including:
memory (29) containing a first set of parameters and a second set of parameters; and
a control processor (28) operable to access from the memory the first set of parameters corresponding to a first operating mode and a second set of parameters corresponding to a second operating mode, wherein the control processor is configured to operate in the first operating mode when the implantable medical device is outside the disruptive energy field and to operate in the second operating mode when the implantable medical device is in the disruptive energy field; said system further comprising:
a programmer that is capable of bi-directional communication with the implantable medical device; **characterized by** the programmer having an application executing thereon that performs an MRI compatible certification process to perform a number of safety checks to ensure that the implantable medical device is MRI compatible before enabling operation in said second operating mode.

2. The implantable medical device of claim 1, further comprising:
a sensor (26) capable of detecting the disruptive energy field and configured to alert the control processor to the presence of the disruptive energy field.

3. The implantable medical device of claim 1, further comprising:
a communication system (30) for communicating with an external device, the external device capable of adjusting the operating mode of the implantable medical device.

4. The implantable medical device of claim 1, wherein the control processor is capable of executing a temporary operation to cause the control processor to operate in the second operating mode, the temporary operation overriding the first operating mode.

5. The implantable medical device of claim 4, wherein the temporary operation causes the implantable medical device to temporarily suspend a device operation of the first operating mode.

6. The implantable medical device of claim 4, wherein the temporary operation suspends collection of diagnostic data.

7. The implantable medical device of claim 1, further comprising:
a magnetic sensor for sensing the presence of the disruptive energy field.

8. The implantable medical device of claim 1, further comprising:
protection electronics (44) for protecting the implantable medical device while exposed to the disruptive energy field.

9. The implantable medical device of claim 8, further comprising:
leads extending from the protection electronics.

10. The system of any one of claim 1-9, wherein the application executing on the programmer checks to see whether an Effective Replacement Indicator (ERI) condition has been met and, if the implantable medical device is at ERI, the application displays a message indicating that the implantable medical device is at ERI.

11. The system of any one of claims 1-10, wherein the application checks whether the impedance of any leads is out of range and, if the measured values are outside of this range, the application displays an error message that the lead impedance of at least one of the leads is out of range.

12. The system of claim 11, wherein the application checks the last recorded lead impedance measurements within the implantable medical device or instructs the implantable medical device to perform a lead impedance check to obtain the lead impedance of the leads.

13. The system of any one of claims 1-12, wherein the application executing on the programmer automatically performs the MRI compatible certification process in response to input from a user selecting an MRI option to begin the process of enabling the MRI exposure mode.

## Patentansprüche

1. System, mit:
einer implantierbaren medizinischen Vorrichtung, die in einem disruptiven Energiefeld betreibbar ist, wobei die implantierbare medizinische Vorrichtung enthält:
Speicher (29), der eine erste Menge von Parametern und eine zweite Menge von Parametern enthält; und
einen Steuerprozessor (28), der betreibbar ist, um auf die erste Menge von Parametern, die einer ersten Betriebsart entspricht, und auf eine zweite Menge von Parametern, die einer zweiten Betriebsart entspricht, in dem Speicher zuzugreifen, wobei der Steuerprozessor konfiguriert ist, um in der ersten Betriebsart zu arbeiten, wenn sich die implantierbare medizinische Vorrichtung außerhalb des disruptiven Energiefeldes befindet, und um in der zweiten Betriebsart zu arbeiten, wenn sich die implantierbare medizinische Vorrichtung in dem disruptiven Energiefeld befindet; wobei das System ferner enthält:
eine Programmiereinrichtung, die zu einer bidirektionalen Kommunikation mit der implantierbaren medizinischen Vorrichtung in der Lage ist; **dadurch gekennzeichnet, dass** die Programmiereinrichtung eine Anwendung besitzt, die darin ausgeführt wird und die einen mit MRI kompatiblen Zertifizierungsprozess ausführt, um zahlreiche Sicherheitsprüfungen auszuführen, um sicherzustellen, dass die implantierbare medizinische Vorrichtung MRI-kompatibel ist, bevor der Betrieb in der zweiten Betriebsart freigegeben wird.

2. Implantierbare medizinische Vorrichtung nach Anspruch 1, ferner mit:
einem Sensor (26), der das disruptive Energiefeld detektieren kann und konfiguriert ist, um den Steuerprozessor vor dem Vorhandensein des disruptiven Energiefeldes zu warnen.

3. Implantierbare medizinische Vorrichtung nach Anspruch 1, ferner mit:
einem Kommunikationssystem (30), um mit einer externen Vorrichtung zu kommunizieren, wobei die externe Vorrichtung die Betriebsart der implantierbaren medizinischen Vorrichtung einstellen kann.

4. Implantierbare medizinische Vorrichtung nach Anspruch 1, wobei der Steuerprozessor eine temporäre Operation ausführen kann, um den Steuerprozessor zu veranlassen, in der zweiten Betriebsart zu arbeiten, wobei die temporäre Operation die erste Betriebsart außer Kraft setzt.

5. Implantierbare medizinische Vorrichtung nach Anspruch 4, wobei die temporäre Operation die implantierbare medizinische Vorrichtung dazu veranlasst, die Operation der Vorrichtung der ersten Betriebsart vorübergehend zu unterbrechen.

6. Implantierbare medizinische Vorrichtung nach Anspruch 4, wobei die temporäre Operation das Sammeln von Diagnosedaten unterbricht.

7. Implantierbare medizinische Vorrichtung nach Anspruch 1, ferner mit:
einem Magnetsensor, um das Vorhandensein des disruptiven Energiefeldes zu erfassen.

8. Implantierbare medizinische Vorrichtung nach Anspruch 1, ferner mit:
Schutzelektronik (44), um die implantierbare medizinische Vorrichtung zu schützen, während sie dem disruptiven Energiefeld ausgesetzt ist.

9. Implantierbare medizinische Vorrichtung nach Anspruch 8, ferner mit:
Leitungen, die sich von der Schutzelektronik erstrecken.

10. System nach einem der Ansprüche 1-9, wobei die Anwendung, die in der Programmiereinrichtung ausgeführt wird, prüft, ob eine Bedingung eines effektiven Austauschindikators (ERI-Bedingung) erfüllt ist, wobei die Anwendung dann, wenn die implantierbare medizinische Vorrichtung der ERI-Bedingung unterliegt, eine Nachricht anzeigt, die angibt, dass die implantierbare medizinische Vorrichtung einer ERI-Bedingung unterliegt.

11. System nach einem der Ansprüche 1-10, wobei die Anwendung prüft, ob die Impedanz irgendwelcher Leitungen außerhalb eines Bereichs liegt, wobei dann, wenn die gemessenen Werte außerhalb des Bereichs liegen, die Anwendung eine Fehlernachricht anzeigt, dass die Leitungsimpedanz wenigstens einer der Leitungen außerhalb des Bereichs liegt.

12. System nach Anspruch 11, wobei die Anwendung die zuletzt aufgezeichneten Leitungsimpedanzmessungen in der implantierbaren medizinischen Vorrichtung prüft oder die implantierbare medizinische Vorrichtung anweist, eine Leitungsimpedanzprüfung auszuführen, um die Leitungsimpedanz der Leitungen zu erhalten.

13. System nach einem der Ansprüche 1-12, wobei die Anwendung, die in dem Programmierer ausgeführt wird, den MRI-kompatiblen Zertifizierungsprozess in Reaktion auf die Eingabe von einem Anwender, die eine MRI-Option auswählt, um den Prozess der Freigabe des MRI-Expositionsmodus zu beginnen, automatisch ausführt.

## Revendications

1. Système comportant :
un dispositif médical implantable opérationnel dans un champ d'énergie disruptive, le dispositif médical implantable incluant :
une mémoire (29) contenant un premier ensemble de paramètres et un second ensemble de paramètres ; et
un processeur de commande (28) opérationnel pour accéder à partir de la mémoire au premier ensemble de paramètres correspondant à un premier mode de fonctionnement et à un second ensemble de paramètres correspondant à un second mode de fonctionnement, dans lequel le processeur de commande est configuré pour fonctionner dans le premier mode de fonctionnement lorsque le dispositif médical implantable se trouve à l'extérieur du champ d'énergie disruptive et pour fonctionner dans le second mode de fonctionnement lorsque le dispositif médical implantable se trouve dans le champ d'énergie disruptive ; ledit système comportant en outre :
un programmateur lequel est capable d'établir une communication bidirectionnelle avec le dispositif médical implantable ; **caractérisé en ce que** le programmateur a une application s'exécutant sur celle-ci qui met en oeuvre un processus de certification compatible avec l'imagerie par résonance magnétique (IRM) pour exécuter un certain nombre de contrôles de sécurité afin de garantir que le dispositif médical implantable est compatible avec l'IRM avant de permettre le fonctionnement dans ledit second mode de fonctionnement.

2. Dispositif médical implantable selon la revendication 1, comportant en outre :
un capteur (26) capable de détecter le champ d'énergie disruptive et configuré pour alerter le processeur de commande de la présence du champ d'énergie disruptive.

3. Dispositif médical implantable selon la revendication 1, comportant en outre :
un système de communication (30) pour communiquer avec un dispositif externe, le dispositif externe pouvant régler le mode de fonctionnement du dispositif médical implantable.

4. Dispositif médical implantable selon la revendication 1, dans lequel le processeur de commande est capable d'exécuter un fonctionnement temporaire pour amener le processeur de commande à fonctionner dans le second mode de fonctionnement, le fonctionnement temporaire remplaçant le premier mode de fonctionnement.

5. Dispositif médical implantable selon la revendication 4, dans lequel le fonctionnement temporaire amène le dispositif médical implantable à suspendre temporairement un fonctionnement du dispositif dans le premier mode de fonctionnement.

6. Dispositif médical implantable selon la revendication 4, dans lequel le fonctionnement temporaire suspend la collecte de données de diagnostic.

7. Dispositif médical implantable selon la revendication 1, comportant en outre :
un capteur magnétique pour détecter la présence du champ d'énergie disruptive.

8. Dispositif médical implantable selon la revendication 1, comportant en outre :
une unité électronique de protection (44) pour protéger le dispositif médical implantable lorsqu'il est exposé au champ d'énergie disruptive.

9. Dispositif médical implantable selon la revendication 8, comportant en outre :
des dérivations qui s'étendent à partir de l'unité électronique de protection.

10. Système selon l'une quelconque des revendications 1 à 9, dans lequel l'application s'exécutant sur le programmateur effectue un contrôle pour voir si une condition d'indicateur de remplacement efficace (ERI) est satisfaite et, si le dispositif médical implantable est à ERI, l'application affiche un message indiquant que le dispositif médical implantable est à ERI.

11. Système selon l'une quelconque des revendications 1 à 10, dans lequel l'application contrôle si l'impédance de l'une quelconque des dérivations est à l'extérieur de la plage et, si les valeurs mesurées sont à l'extérieur de cette plage, l'application affiche un message d'erreur indiquant que l'impédance de dérivation d'au moins l'une des dérivations est à l'extérieur de la plage.

12. Système selon la revendication 11, dans lequel l'application contrôle les dernières mesures d'impédance de dérivation enregistrées à l'intérieur du dispositif médical implantable ou ordonne au dispositif médical implantable d'exécuter un contrôle d'impédance de dérivation afin d'obtenir l'impédance de dérivation des dérivations.

13. Système selon l'une quelconque des revendications 1 à 12, dans lequel l'application s'exécutant sur le programmateur met en oeuvre automatiquement le processus de certification compatible avec l'IRM en réponse à l'entrée d'un utilisateur sélectionnant une option IRM pour commencer le processus d'activation du mode d'exposition IRM.
